# EUROPEAN PATENT APPLICATION

(11) **EP 4 556 054 A2**
(43) Date of publication of application: **21.05.2025**
(21) Application number: 25167887.6
(22) Date of filing: 29.03.2021
(51) Int. Cl.: A61N 1/05

(54) **METHOD FOR MANUFACTURING A COCHLEAR IMPLANT ELECTRODE LEAD**

(62) Divisional of application: 21165630.1
(71) Applicant: Advanced Bionics AG, 8712 Staefa (CH)
(72) Inventor: GOMMEL, Uli, Valencia, 91355 (US); PEREZ, Martin Sandoval, Canyon Country, 91387 (US); SMITH, James, Santa Clarita, 91350 (US); WISE, Nicolas, Pasadence, 91106 (US); GANDARIA, Enrique, Santa Clarita, 91350 (US)
(74) Representative: Schwan Schorer & Partner mbB

(57) **Abstract**

There is provided an electrode lead for a cochlear implant system, comprising a tube (150, 152, 154) having an interior volume (158) and an outer peripheral surface (159); a plurality of stimulating electrodes (112) disposed on a distal portion of the electrode lead (110); a ground electrode (120) disposed on a proximal portion of the electrode lead for providing a current return path for stimulation current generated by the plurality of stimulation electrodes, wherein the ground electrode is ring-shaped and is fixed on and around the outer peripheral surface of the tube; and a ground wire (160) extending within the interior volume of the tube to the ground electrode and being electrically connected to the ground electrode through an opening (156) in a wall of the tube. The ground electrode comprises a first end portion (122), a central portion (124) axially adjacent to the first end portion, and a second end portion (126) axially adjacent to the central portion, wherein the outer surface (128) of the central portion is radially elevated relative to the outer surface (130) of the first end portion and the outer surface of the second end portion (134), so as to form a step (134, 136) at the boundary between the first end portion and the central portion and at the boundary between the second end portion and the central portion, respectively.

## Description

The invention relates to an electrode lead for a cochlear implant system and a method for manufacturing the same.

Cochlear implant systems are used to provide, restore and/or improve hearing loss suffered by cochlear implant patients who use the cochlear implant systems. A key component of a cochlear implant system is an electrode lead that is inserted into a cochlea of the patient. A distal end portion of the electrode lead is provided with a plurality of stimulation electrodes for applying a stimulation current to auditory nerve tissue within the cochlea, which is supplied via electrode wires extending through the electrode lead and electrically connecting the respective stimulation electrode with a cochlear implant implanted within the patient at the proximal end of the electrode lead. A proximal portion of the electrode lead may be provided with one or more ground electrodes for providing a current return path for stimulation current applied by the stimulation electrodes; alternatively, the ground electrode(s) may be provided separate from the electrode lead. The ground electrode may be designed as a ring electrode, which is disposed on the electrode lead and is electrically connected via a ground wire extending within the proximal portion of the electrode lead to the cochlear implant. An example of such cochlear implant system is described in WO 2019/045747 A1.

Another example of a cochlear implant electrode lead with a ground ring electrode is known from US 9,561,361 B1, wherein the ring electrode is provided on a large diameter tube, with the stimulation electrode wires being helically coiled through the interior of the tube which is backfilled with silicone; also the ground wire is helically coiled and passes through the center of the helically coiled stimulation wires. Each side of the ring electrode is provided with silicone tapers for smoothing the profile of the tube, for securing the ring electrode in place and for reducing the potential for kinking of the tube. The ring electrode has the shape of a hollow cylindrical cylinder. The ground wire passes out of an opening in the sidewall of the tube and is electrically connected to the ring electrode disposed over the tube.

The ring of the ground electrode and the ground wire may be susceptible to fluid ingress, especially if the electrode lead has been stressed near the ring or the device fantail; fluid ingress can lead to high electrode impedances over time. Further, silicone tapers at both sides of the ring electrode - due to lack of a consistent boundary of the applied silicone - may be susceptible to variants of silicone application.

It is an object of the invention to provide for an electrode lead for a cochlear implant system, having a ground electrode which is relatively resistant to fluid ingress and which can be manufactured in a relatively consistent and efficient manner. It is a further object to provide for a method of manufacturing such electrode lead.

According to the invention, these objects are achieved by an electrode lead as defined in claim 1 or claim 14 and a manufacturing method as defined in claim 15, respectively.

The electrode lead of claim 1 is beneficial in that, by providing the ground ring electrode with a central portion whose outer surface is radially elevated relative to the outer surface of the adjacent first end portion and second end portion of the ground ring electrode, so as to form a step at the boundary between the central portion and the first end portion and the second end portion, respectively, or by providing the end portion of the ground ring electrode with ring-like marker structures, reliability and manufacturability of the electrode lead can be improved. In particular, the step or the marker structure provides for a boundary of the silicone application to the end portions of the ground ring electrode, thereby improving consistency of silicone application for the silicone coating of the end portions of the ground ring electrode and the adjacent outer surface of the tube.

According to one embodiment, the outer diameter of the central portion of the ground electrode may be axially substantially constant over the entire length of the central portion so that the outer surface of the central portion is substantially cylindrical.

According to one embodiment, the outer diameter of the first end portion of the ground electrode may be axially substantially constant over the entire length of the first end portion, and the outer diameter of the second end portion of the ground electrode may be axially substantially constant over the entire length of the second end portion.

According to one embodiment, the first end portion and the second end portion of the ground electrode may have substantially the same outer diameter.

According to one embodiment, the first end portion and second end portion of the ground electrode and the central portion of the ground electrode may all have the same inner diameter.

According to one embodiment, the axial dimension of the first end portion of the ground electrode may be substantially identical to the axial dimension of the second end portion of the ground electrode.

According to one embodiment, the ground electrode may comprise at least one slot for receiving an end portion of the ground wire for attachment of the ground wire to the ground electrode.

According to one embodiment, the at least one slot may be provided on the edge of the first end portion and/or the second end portion of the ground electrode, or the at least one slot may be provided in the central portion of the ground electrode.

According to one embodiment, the first end portion and the second end portion of the ground electrode each may be covered by a silicone coating which extends from the outer surface of the tube to the step at the boundary between the central portion and the respective first end portion or the second end portion of the ground electrode so as to seal to the ground electrode to the tube.

According to one embodiment, the outer surface of each of the silicone coatings may substantially flush with the outer surface of the central portion of the ground electrode.

According to one embodiment, the first end portion and/or the second end portion of the ground electrode may comprise at least one opening for ingress of silicone for anchoring the silicone coating to the respective first or second end portion of the ground electrode.

According to one embodiment, the at least one opening may comprise at least one slot provided on the edge of the first end portion and/or the second end portion, or the at least one opening may comprise at least one hole.

According to one embodiment, the outer surface of the first end portion and/or the second end portion of the ground electrode may be provided with surface structures, such as circumferential ribs or circumferential groves forming a partial thread, for improving adhesion of the silicone coating.

According to one embodiment, the outer surface of the first end portion and/or the second end portion of the ground electrode may be roughened for improving adhesion of the silicone coating.

According to one embodiment, the step at the boundary between the first end portion and the central portion and/or the step at the boundary between the second end portion and the central portion may be provided with recesses in the outer surface of the central portion extending axially into the part of the central portion elevated with regard to the first and second end portion of the ground electrode, and the recesses may be configured to allow ingress of silicone for anchoring the silicone coating of the respective first or second end portion at the step.

According to one embodiment, at least part of the inner surface of the ground electrode may comprise a silicone adhesive layer sandwiched between the outer surface of the tube and the inner surface of the ground electrode.

According to one embodiment, the ground wire may be coated with PPSU.

According to one embodiment, electrode wires for the plurality of stimulation electrodes may extend through the interior volume of the tube in a helically wound configuration, and the ground wire may be co-radially wound with regard to the electrode wires.

According to one embodiment, the tube may comprise a first tube portion and a second tube portion which may be axially aligned to each other and which may axially abut each other, thereby forming a circumferential gap in-between which is filled with silicone and which is surrounded by the ground electrode.

According to one embodiment, the gap may form the opening of the tube through which the ground wire extends to the ground electrode.

According to one embodiment, the interior volume of the tube may be backfilled with a fast-cure silicone adhesive.

According to one embodiment, the outer surface of the central portion may be polished or laser textured.

According to one embodiment, the ground electrode may be made of Pt, Ti, a PtIr alloy or a Ti alloy.

According to one embodiment, the ratio of the outer surface area of the central portion of the ground electrode to the total outer surface area of the ground electrode may be from 0.50 to 0.75.

According to one embodiment of the electrode lead of claim 14, each of the ring-like marker structures may comprise two parallel rings which may be axially spaced apart. for example, each of the ring-like marker structures may be formed by an engraved or laser treated surface portion.

The manufacturing method defined in claim 15 is beneficial in that it allows for particularly consistent and efficient manufacturing of the electrode lead. In particular, the dual tube configuration, with a first tube portion and a second tube portion abutting each other within the interior of the ground ring electrode, allows for full assembly of a sub-assembly of the ground ring electrode and the ground wire attached to the ground ring electrode, with the ground wire extending through the gap between the first tube portion and the second tube portion, whereby the need for passing the ground wire through the inner diameter of the tub and pulling it out through an opening cut in the tube wall can be avoided.

According to one embodiment, the ground wire may be wound co-radially with the electrode wires. This improves manufacturability and decreases potential-process failures.

According to one embodiment, the ground wire may be made of Pt and coated with PPSU, whereby the bonding to the ground wire and a silicone backfill of the tube may be improved.

According to one embodiment of the method of claim 15, the outer surface of the central portion of the ground electrode is radially elevated relative to the outer surface of the first end portion and the outer surface of the second end portion of the ground electrode, so as to form a step at the boundary between the first end portion and the central portion and at the boundary between the second end portion and the central portion, respectively, and the silicone coating is applied to the outer surface of the first and second end portion of the ground electrode in such a manner that the outer surface of each of the silicone coatings is substantially flush with the outer surface of the central portion of the ground electrode.

According to one embodiment of the method of claim 15, each of the first end portion and the second end portion comprises a ring-like marker structure, and the silicone coating is applied to the outer surface of the first and second end portion of the ground electrode in such a manner that the silicone coating does not extend axially beyond the ring-like marker structures into the central portion.

According to one embodiment of the method of claim 15, the ground wire is wound co-radially with the electrode wires onto the mandrel.

Preferred embodiments of the invention are defined in the dependent claims.

Hereinafter, examples of the invention will be illustrated by reference to the attached drawings, wherein:
- Fig. 1: schematically illustrates an example of a cochlear implant system;
- Fig. 2: shows a partially cut-away perspective view of an example of a ground ring electrode mounted on a cochlear lead;
- Fig. 3: shows a cross-sectional view of the ground electrode of Fig. 2;
- Fig. 4: is a view similar to Fig. 2, wherein only the ground ring electrode - but not the tube - is shown partially cut away;
- Figs. 5A to 5J: show different examples of ground ring electrodes;
- Figs. 6A to 6F: illustrate steps of an example of an assembly process of a cochlear lead with a ground ring electrode;
- Fig. 7: illustrates a dual tube concept used in the manufacturing process of a cochlear lead with a ground ring electrode;
- Fig. 8: is a side view of an example of a cochlear implant with an electrode lead: and
- Fig. 9: shows an alternative example of a ground ring electrode.

As used hereinafter, the term "distal" refers to portions or components that are farther away from the cochlear implant (hermitically sealed enclosure) or the surgeon implanting the cochlear implant, and the term "proximal" refers to portions or components that are closer to the cochlear implant or the surgeon implanting the cochlear implant. As used hereinafter, the term "substantially" should be understood as "within ± 10%".

The terms "ground electrode", "ring electrode" and "ground ring electrode" are used in an interchangeable manner hereinafter to designate a ring-shaped ground electrode.

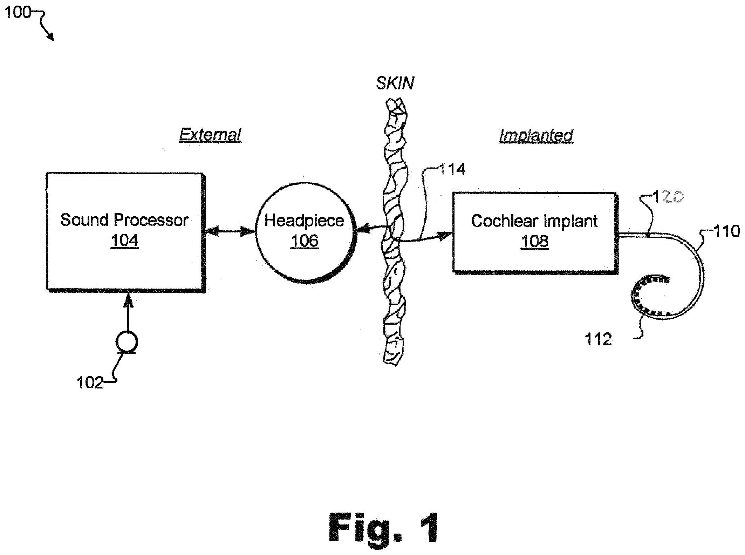

FIG. 1 illustrates an exemplary cochlear implant system 100. As shown, cochlear implant system 100 may include a microphone 102, a sound processor 104, a headpiece 106 having a coil disposed therein, a cochlear implant 108, and an electrode lead 110.

Electrode lead 110 includes an array of stimulating electrodes 112 (also referred to as intracochlear electrodes) disposed on a distal portion of electrode lead 110 and that are configured to be located within and to stimulate the cochlea after the distal portion of electrode lead 110 is inserted into the cochlea. As shown, electrode lead 110 also includes a ground electrode 120 (also referred to as a ring electrode) disposed on a proximal portion of electrode lead 110 and that is configured provide a current return path for stimulation current generated by stimulation electrodes 112 and to remain external to the cochlea after electrode lead 110 is inserted into the cochlea. While a single ground electrode 120 is shown in FIG. 1, it will be recognized that multiple ground electrodes 120 may be disposed on the proximal portion of electrode lead 110 as may serve a particular implementation. As shown, electrode lead 110 may be pre-curved so as to properly fit within the spiral shape of the cochlea. Additional or alternative components may be included within cochlear implant system 100 as may serve a particular implementation.

As shown, cochlear implant system 100 may include various components configured to be located external to a patient including, but not limited to, microphone 102, sound processor 104, and headpiece 106. Cochlear implant system 100 may further include various components configured to be implanted within the patient including, but not limited to, cochlear implant 108 and electrode lead 110.

Microphone 102 may be configured to detect audio signals presented to the user. Microphone 102 may be implemented in any suitable manner. For example, microphone 102 may include a microphone that is configured to be placed within the concha of the ear near the entrance to the ear canal, such as a T-MIC^{™} microphone from Advanced Bionics. Such a microphone may be held within the concha of the ear near the entrance of the ear canal by a boom or stalk that is attached to an ear hook configured to be selectively attached to sound processor 104. Additionally, or alternatively, microphone 102 may be implemented by one or more microphones disposed within headpiece 106, one or more microphones disposed within sound processor 104, one or more beam-forming microphones, and/or any other suitable microphone as may serve a particular implementation.

Sound processor 104 (i.e., one or more components included within sound processor 104) may be configured to direct cochlear implant 108 to generate and apply electrical stimulation (also referred to herein as "stimulation current") representative of one or more audio signals (e.g., one or more audio signals detected by microphone 102, input by way of an auxiliary audio input port, input by way of a clinician's programming interface ("CPI") device, etc.) to one or more stimulation sites associated with an auditory pathway (e.g., the auditory nerve) of the patient. Exemplary stimulation sites include, but are not limited to, one or more locations within the cochlea, the cochlear nucleus, the inferior colliculus, and/or any other nuclei in the auditory pathway. To this end, sound processor 104 may process the one or more audio signals in accordance with a selected sound processing strategy or program to generate appropriate stimulation parameters for controlling cochlear implant 108. Sound processor 104 may be housed within any suitable housing (e.g., a behind-the-ear ("BTE") unit, a body worn device, headpiece 106, and/or any other sound processing unit as may serve a particular implementation).

In some examples, sound processor 104 may wirelessly transmit stimulation parameters (e.g., in the form of data words included in a forward telemetry sequence) and/or power signals to cochlear implant 108 by way of a wireless communication link 114 between headpiece 106 and cochlear implant 108 (e.g., a wireless link between a coil disposed within headpiece 106 and a coil physically coupled to cochlear implant 108). It will be understood that communication link 114 may include a bi-directional communication link and/or one or more dedicated uni-directional communication links.

Headpiece 106 may be communicatively coupled to sound processor 104 and may include an external antenna (e.g., a coil and/or one or more wireless communication components) configured to facilitate selective wireless coupling of sound processor 104 to cochlear implant 108. Headpiece 106 may additionally or alternatively be used to selectively and wirelessly couple any other external device to cochlear implant 108. To this end, headpiece 106 may be configured to be affixed to the patient's head and positioned such that the external antenna housed within headpiece 106 is communicatively coupled to a corresponding implantable antenna (which may also be implemented by a coil and/or one or more wireless communication components) included within or otherwise associated with cochlear implant 108. In this manner, stimulation parameters and/or power signals may be wirelessly transmitted between sound processor 104 and cochlear implant 108 via a communication link 114 (which may include a bi-directional communication link and/or one or more dedicated uni-directional communication links as may serve a particular implementation).

Cochlear implant 108 may include any type of implantable stimulator that may be used in association with the systems and methods described herein. For example, cochlear implant 108 may be implemented by an implantable cochlear stimulator. In some alternative implementations, cochlear implant 108 may include a brainstem implant and/or any other type of cochlear implant that may be implanted within a patient and configured to apply stimulation to one or more stimulation sites located along an auditory pathway of a patient.

In some examples, cochlear implant 108 may be configured to generate electrical stimulation representative of an audio signal processed by sound processor 104 (e.g., an audio signal detected by microphone 102) in accordance with one or more stimulation parameters transmitted thereto by sound processor 104. Cochlear implant 108 may be further configured to apply the electrical stimulation to one or more stimulation sites (e.g., one or more intracochlear regions) within the patient via electrodes 112, 120 disposed along electrode lead 110 (e.g., applying current by way of stimulating electrodes 112 that returns by way of ground electrode 120). In some examples, cochlear implant 108 may include a plurality of independent current sources each associated with a channel defined by one or more of electrodes 112. In this manner, different stimulation current levels may be applied to multiple stimulation sites simultaneously by way of multiple electrodes 112.

The human cochlea is in the shape of a spiral beginning at a base and ending at an apex, and auditory nerve tissue resides within the cochlea resides. The auditory nerve tissue is organized within the cochlea in a tonotopic manner. Relatively low frequencies are encoded at or near the apex of the cochlea (referred to as an "apical region") while relatively high frequencies are encoded at or near the base (referred to as a "basal region"). Hence, electrical stimulation applied by way of electrodes disposed within the apical region (i.e., "apical electrodes") may result in the patient perceiving relatively low frequencies and electrical stimulation applied by way of electrodes disposed within the basal region (i.e., "basal electrodes") may result in the patient perceiving relatively high frequencies. The delineation between the apical and basal electrodes on a particular electrode lead may vary depending on the insertion depth of the electrode lead, the anatomy of the patient's cochlea, and/or any other factor as may serve a particular implementation.

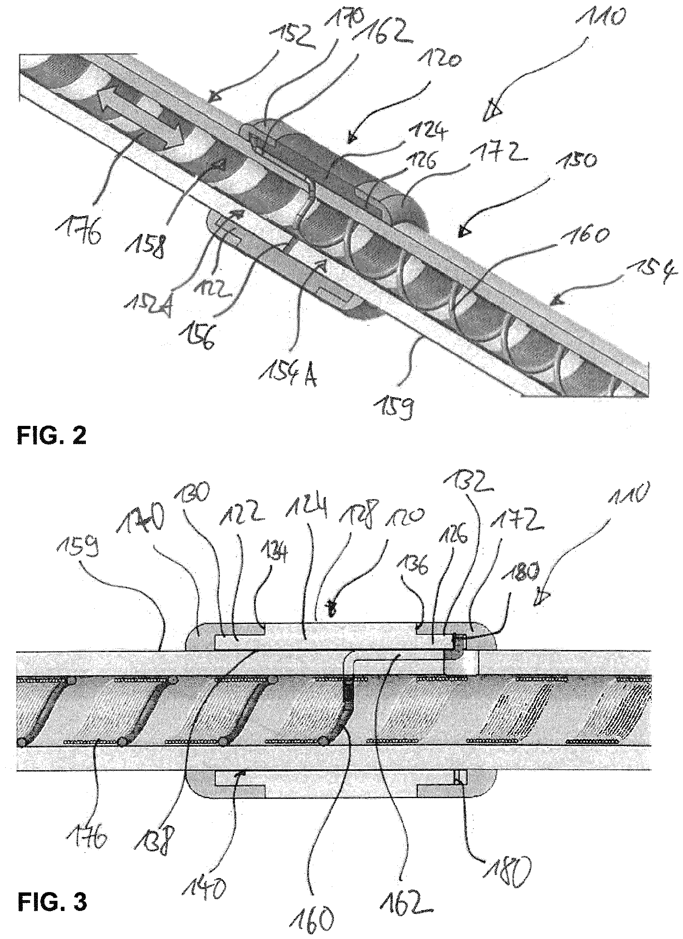

An example of a ring-shaped ground electrode (hereinafter "ring electrode 120") disposed on the electrode lead 110 is shown in Figs. 2 and 3. The electrode lead 110 comprises a tube 150 having a first tube portion 152 and a second tube portion 154, which are axially aligned to each other and which axially abut each other, thereby forming a circumferential gap 156, which is filled with silicone adhesive and which is surrounded by the ring electrode 120. The tube 150 may be formed, for example, from silicone with a shore A hardness of approximately 30 to 70. The tube 150 has an interior volume 158, which is backfilled with a fast-cure silicone adhesive, such as an adhesive available under the designation NuSil Technology MED2-4213 from the company Avantor located in Radnor, Pennsylvania, USA.

A helically wound or coiled ground wire 160 extends from the cochlear implant 108 through the interior volume 158 of the second tube portion 154 to the ring electrode 120, to which it is electrically connected, typically via laser welding. As can be seen in Fig. 2, an end portion 162 of the ground wire 160 extends through the gap 156, which thus forms an opening in the tube 150, to the ring electrode 160. The ground wire 160, in one example, may be coated with PPSU and may be made of Pt. This provides for a good bonding between the ground wire 160 and the silicone backfill in the interior volume 158 of the tube 150. A backfill of with a silicone adhesive, such as one available under the designation NuSil Technology MED2-4213 from the company Avantor located in Radnor, Pennsylvania, USA, in the interior volume 158 provides for a good bonding to the PPSU coated ground wire 160 and the wall of the tube 150. In some implementations, the ground wire may be solid; alternatively, it may be multi-stranded.

The preferred materials for the coating of the ground wire 160 and the backfill of the tube 150 provide for good fluid resistance and increased adhesion.

As shown in Figs. 2 and 3, a plurality of electrode wires 176 extends in a helically wound/coiled configuration through the backfilled interior volume 158 of the tube 150, wherein each of the electrodes wires 176 connects one of the stimulation electrodes 112 to the cochlear implant 108. As can be seen in Figs. 2 and 3, the ground wire 160 is co-radially wound with regard to the electrode wires 176. Typically, the ground wire 160 has a larger diameter than each one of the electrode wires 176, so that it is able to carry the sum of the stimulation currents produced by the electrodes 112. The ground wire 160 may be multi-strand to reduce the stiffness resulting from the larger diameter.

The ring electrode 120 comprises a first end portion 122, a central portion 124 axially adjacent to the first end portion 122 and a second end portion 126 axially adjacent to the central portion 124. The outer surface 128 of the central portion 124 is radially elevated relative to the outer surface 130 of the first end portion 122 and the outer surface 132 of the second end portion 126, so as to form a step 134 at the boundary between the first end portion 122 and the second end portion 124 and a step 136 at the boundary between the second end portion 126 and the central portion 124, respectively. The outer surface 128 of the central portion 124 may be highly polished for best biofilm resistance or it may be laser textured to increase the surface area.

In the example shown in Figs. 2 to 4, the outer diameter of the central portion 124 of the ring electrode 120 is axially substantially constant over the entire length of the central portion 124, so that the outer surface 128 of the central portion 124 is substantially cylindrical. Also, the outer diameter of the first end portion 122 and the second end portion 126 is substantially constant over the entire length of the respective end portion, so that the outer surface 130, 132 of the first end portion 122 and the second end portion 126 is substantially cylindrical, with the first end portion 122 and the second end portion 124 having substantially the same outer diameter. Further, the ring electrode 120 may be symmetric in the axial direction, i.e., the axial dimension of the first end portion 120 is substantially the same as the axial dimension of the second end portion. Also, the first end portion 122, the second end portion 126 and the central portion 124 may have the same inner diameter, so that the inner surface 138 of the ring electrode 120 is circular cylindrical.

The ring electrode 120 may be made of Pt, Ti, a Ptlr alloy or a Ti alloy or any other biocompatible conductor.

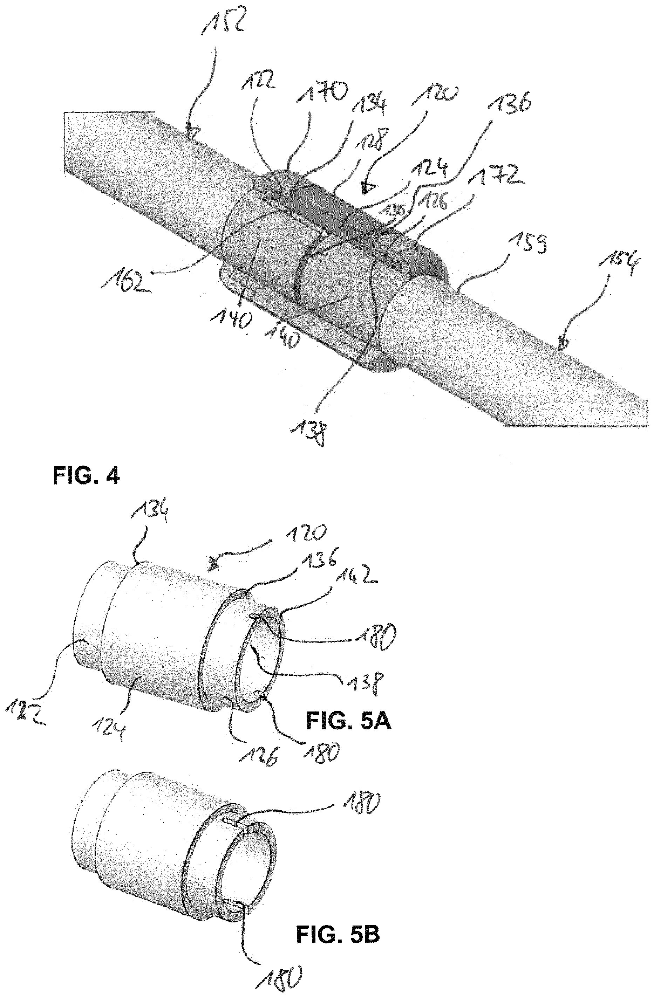

As can be seen in Fig. 4, at least part of the inner surface 138 of the ring electrode is provided with a silicone adhesive coating 140, such as one available under the designation NuSil Technology MED3-4213 from the company Avantor located in Radnor, Pennsylvania, USA, which is located between the outer surface 159 of the tube 150 and the inner surface 138 of the ring electrode 120, so as to attach the ring electrode 120 to the tube 150.

As can be seen in Figs. 2 to 4, the first end portion 122 and the second end portion 126 of the ring electrode 120 are covered, on the respective outer surface 130, 132, by a silicone coating (which also may be labelled "taper") 170, 172, which extends from the outer surface 159 of the tube 150 to the step 134, 136 at the boundary between the central portion 124 and the respective first end portion 122 or second end portion 126. The silicone coatings 170, 172 serve to stabilize attachment of the ring electrode 120 to the tube 150 and to seal the edges 142,144 of the first and second end portions 170, 172 to the tube 150, so as to prevent ingress of fluid into the space between the inner surface 138 of the ring electrode 120 and the outer surface 159 of the tube 150. The silicone coatings 170, 172 may be made of, for example, NuSil Technology MED3-4213, like the attachment layer 140 between the tube 150 and the inner surface 138 of the ring electrode 120. In some examples, the ring electrode 120 may be mode of Ti, with a primer compound being used to promote adhesion of the silicone material to the Ti surface of the ring electrode 120.

As can be seen in Figs. 2 to 4, the silicone coatings 170 or 172 are applied in such a manner that the outer surface of each of the silicone coatings 170, 172 is radially substantially flush with the outer surface 128 of the central portion 124 of the ring electrode 120. In particular, the silicone coatings 170, 172 terminate at the steps 134, 136, respectively, so that the entire outer surface 128 of the central portion 124 of the ring electrode 120 remains exposed for contact with tissue when implanted. Thereby, the manufacturing process results in a reproducible and well-defined exposed surface area of the ring electrode 120. In particular, the steps 134, 136 prevent inconsistency of silicone application when producing the silicone coatings 170, 172.

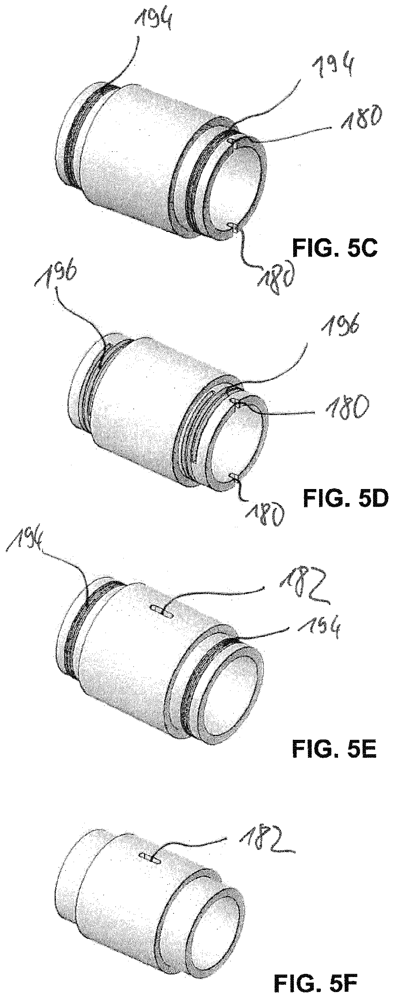

As illustrated by the examples shown in Figs. 5A to 5J, the first end portion 122 and/or the second end portion 124 may be provided with features for attachment of the ground wire 160 to the ring electrode 120 and/or with features for improving adhesion of the silicone coating 170, 172 at the first and second end portions 122, 126. Alternatively, the central portion 124 may be provided with a feature for attachment of the ground wire 160.

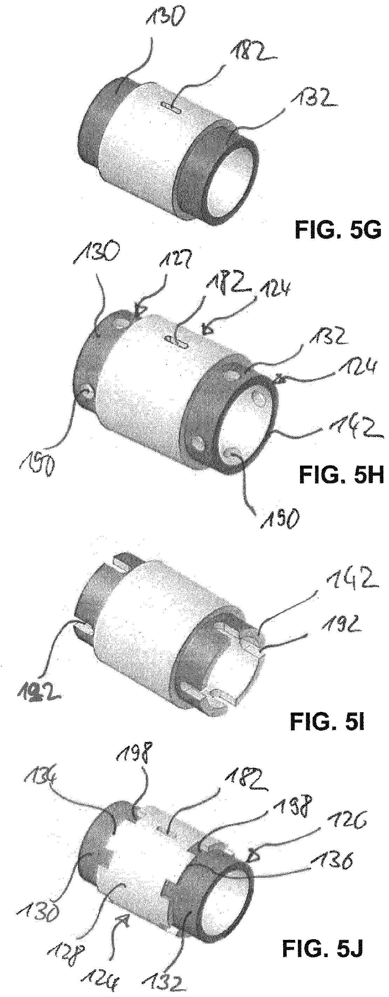

In some embodiments, the ring electrode 120 comprises at least one opening for receiving an end portion 162 of the ground wire 120, which is to be welded to the ground electrode 120. For example, as illustrated in Figs. 5A to 5H and 5J, at least one opening may comprise at least one slot for receiving the end portion 162 of the ground wire 160. As illustrated in Figs. 5A to 5D, at least one slot 180 may be provided on the edge 142 of the second end portion 126. For example, as shown in Figs. 5A to 5D, two slots 180 may be provided on the edge 142, which are circumferentially spaced apart by 180 degrees, so that the two slots 180 are opposite to each other. As can be seen in Fig. 2, the end of the end portion 162 of the ground wire 160 may be inserted into one of the two slots 180, so as to weld, for example by laser welding, the ground wire 160 to the ring electrode 120.

In the examples shown in Figs. 5E to 5H and 5J, the central portion 124 of the ring electrode 120 may be provided with at least one slot 182 for receiving an end of the ground wire 160, so as to weld the ground wire 160 to the ring electrode 120. Also, in these examples, two of the slots 182 may be provided circumferentially spaced apart by 180 degrees (only one of these slots 182 is visible in the Figures).

Various features for enhancing attachment of the silicone coatings 170 or 172 to the ring electrode 120 are illustrated in Figures 5C to 5J.

For example, the first end portion 122 and/or the second end portion 126 of the ring electrode 120 may comprise at least one opening for ingress of silicone during manufacturing for anchoring the silicone coating 170, 172 to the respective first or second end portion 122, 126. For example, as shown in Fig. 5H, the first and second end portion 122, 126 may be provided with a plurality of holes, in particular circular holes 190, which are circumferentially spaced apart. Alternatively, as shown in Fig. 5I, the edges 142, 144 of the first and second end portions 122, 126 may be provided with circumferentially spaced apart slots 192.

In other examples, the outer surface 130, 132 of the first and second end portions 122, 126 are provided with surface structures for improving the adhesion of the silicone coating 170, 172. For example, as shown in Figs. 5C to 5E, circumferential ribs 194 (see Figs. 5C and 5E) or circumferential grooves 196 forming a partial thread may be provided on the outer surfaces 130, 132 of the first and second end portions 122, 126.

Alternatively, or in addition, the outer surfaces 130, 132 of the first and second end portions 122, 126 may be roughened for improving adhesion of the silicone coating 170, 172, such as by bead blast treatment, laser treatment or chemical etch; this is schematically illustrated by the darker color in Figs. 5G to 5J.

In some examples, the step 134, 136 between the first and second end portion 122, 126 and the central portion 124, respectively, may be provided with recesses 198, which are circumferentially spaced apart and which are provided in the outer surface 128 of the central portion 124 and extend axially into the elevated part of the central portion 124. The recesses 198 are configured to allow ingress of silicone during manufacturing for anchoring the silicone coatings 170, 172 in the respective first or second end portion 122, 126 at the steps 134, 136 (see Fig. 5J).

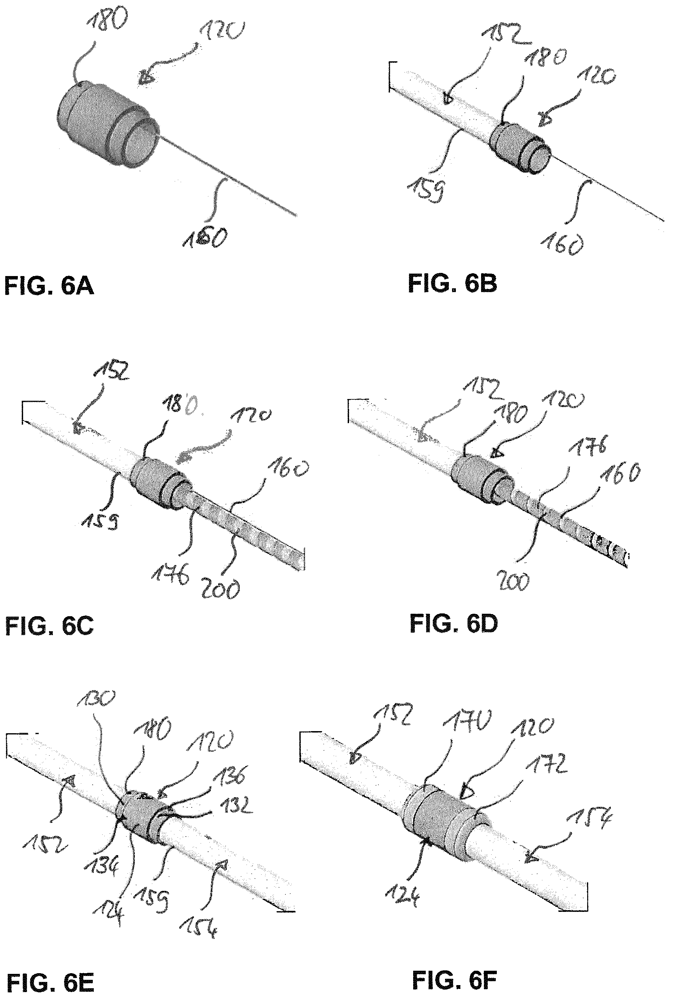

An example of a method for manufacturing the electrode lead 100 illustrated in Figs. 2 to 4 is shown in Figs. 6A to 6F.

In a first step illustrated in Fig. 6A, the ring electrode 120 is provided and an end of a ground wire 160 is electrically connected to the ring electrode 120, for example at a slot 180 (in the method illustrated in Figs. 6A to 6F the ring electrode 120 is of the type shown in Fig. 5A); preferably, a laser welding process is used.

In the example of Fig. 6A the welding slot 180 is provided in the first end portion 122 of the ring electrode 120, and the ground wire 160 extends through the interior volume of the ring electrode 120 out of the second end portion 126.

In the step shown in Fig. 6B the sub assembly formed by the ring electrode 120 and the ground wire welded thereto is fit to one end 152A of a first tube portion 152, wherein first a silicone adhesive, such as NuSil Technology MED3-4213, is applied to the inner surface 138 of the ring electrode 120, so as to form the layer 140 shown in Fig. 4, and the first end portion 122 of the ring electrode is slid over the outer surface 159 of the end 152A of the first tube portion 152, so as to attach the ring electrode 120 to the first tube portion 152. Thereby, the end portion 162 of the ground wire 160 is sandwiched between the outer surface 159 of the first tube 152 and the inner surface 138 of the ring electrode 120, as illustrated in Figs. 2 to 4. After the step shown in Fig. 6B the end 152A of the first tube portion 152 extends into the ring electrode 120 from one side along a part of the axial length of the ring electrode 120.

In a next step, a plurality of parallel stimulation electrode wires 176 is helically wound/coiled onto a mandrel 200. As illustrated in Fig. 6C, the mandrel 200 with the stimulation electrode wires 176 is inserted into the interior of the ring electrode 120 and the interior volume 158 of the first tube portion 152. Thereafter, the ground wire 160 is wound co-radially with the electrode wires 176 onto the mandrel 200.

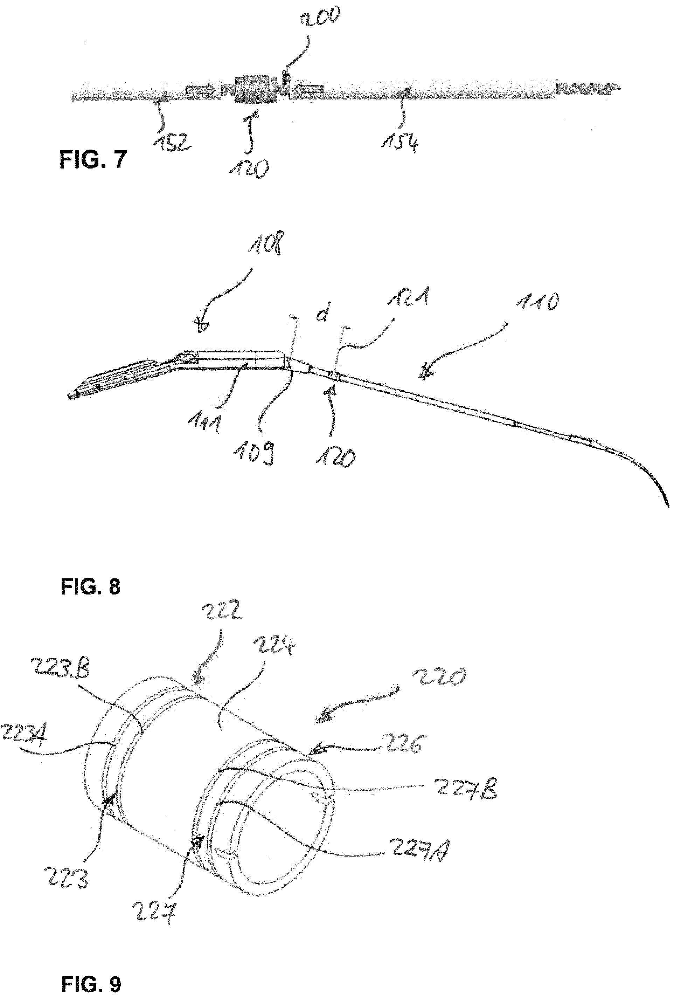

In a next step, as illustrated in Fig. 6E, a second tube portion 154 is slid over the mandrel 200 with the stimulation electrode wires 170 and the ground wire 160, and one end 155A of the second tube portion 154 is inserted into the interior of the ring electrode 120, so that the end 154A of the second tube portion 154 abuts the end 152A of the second tube portion, thereby forming a circumferential gap 156 through which the end portion 162 of the ground wire passes towards the interior surface 138 of the ring electrode 120. In this step, the ring electrode 120 is attached on the second tube portion 154 via silicone adhesive applied to the interior surface 138 of the ground electrode, resulting in the adhesive layer 140. The dual tube concept is also illustrated in Fig 7, wherein it is shown by arrows that the first tube portion 152 and the second tube portion 154 are slid over the mandrel 200 towards each other, so as to meet in the interior of the ring electrode 120.

In the step illustrated in Fig. 6F, silicone coatings 170, 172 are applied to the outer surfaces 130, 132 of the end portions 122, 126 and to the outer surface 159 of the first tube portion 152 and the second tube portion 154 in a region adjacent to the ring electrode 120, so as to seal the ring electrode 120 to the first tube portion 152 and the second tube portion 154. The silicone coatings 170, 172 are applied in such a manner that the outer surfaces of the silicone coatings 170, 172 are flush with the outer surface 128 of the central portion 124 of the ring electrode 120.

In some implementations the ratio of the outer surface area of the central portion 124 to the total outer surface area of the ring electrode 120 (formed by the sum of the outer surfaces of the central portion 124 and the first and second end portions 122, 126) is from 0.50 to 0.75. The outer surface of the central portion 124 represents the exposed surface of the ring electrode 120.

In Fig. 8 a side view of an example of a cochlear implant 108 connected to an electrode lead 110 having a ring electrode 120 is shown, wherein it is illustrated that the distance d in the axial direction between an end 109 of the housing / case 111 of the cochlear implant 108 from which the electrode lead 108 extends and a central cross sectional plane 121 extending through the center of the axial length of the ring electrode 120 (the plane 121 represents the axial center of the ring electrode 120) may be from 8.8 to 14.8 mm to optimize surgical placement of the cochlear implant system and to achieve optimal electrical performance.

While in the above examples of the ring electrode 120 the central portion 124 is elevated so as to ensure proper placement of the silicone coating on the ring electrode120 by providing a boundary of the silicone application to the end portions of the ring electrode, in an alternative embodiment such boundary may be provided by a ring-like marker structure on each of the end portions of the ring electrode. Such ring-like marker structures serve to mark the axial boundary of the proper silicone application, so as to ensure that the operator applies a silicone coating to the outer surface of the first and second end portion of the ground electrode in such a manner that the silicone coating does not extend axially beyond the respective marker structure into the central portion.

An example of such ring electrode 220 is shown in Fig. 9, wherein each of the first end portion 222 and the second end portion 226 comprises a ring-like marker structure 223, 227. In the example of Fig. 9, each ring-like marker structure 223, 227 comprises to parallel axially spaced apart rings 223A, 223B and 227A, 227B, respectively. For example, the ring-like marker structures 223, 227 may be generated by engraving or laser treatment of surface portions of the first end portion 222 and the second end portion 226.

## Claims

1. An electrode lead for a cochlear implant system (100), comprising
a tube (150, 152, 154) having an interior volume (158) and an outer peripheral surface (159);
a plurality of stimulating electrodes (112) disposed on a distal portion of the electrode lead (110);
a ground electrode (120) disposed on a proximal portion of the electrode lead for providing a current return path for stimulation current generated by the plurality of stimulation electrodes, wherein the ground electrode is ring-shaped and is fixed on and around the outer peripheral surface of the tube;
a ground wire (160) extending within the interior volume of the tube to the ground electrode and being electrically connected to the ground electrode through an opening (156) in a wall of the tube;
wherein the ground electrode comprises a first end portion (122), a central portion (124) axially adjacent to the first end portion, and a second end portion (126) axially adjacent to the central portion, wherein the outer surface (128) of the central portion is radially elevated relative to the outer surface (130) of the first end portion and the outer surface of the second end portion (134), so as to form a step (134, 136) at the boundary between the first end portion and the central portion and at the boundary between the second end portion and the central portion, respectively.

2. The electrode lead of claim 1, wherein the outer diameter of the central portion (124) of the ground electrode (120) is axially substantially constant over the entire length of the central portion so that the outer surface (128) of the central portion is substantially cylindrical.

3. The electrode lead of claim 1 or 2, wherein the outer diameter of the first end portion (122) of the ground electrode (120) is axially substantially constant over the entire length of the first end portion, and wherein the outer diameter of the second end portion (126) of the ground electrode is axially substantially constant over the entire length of the second end portion.

4. The electrode lead of one of the preceding claims, wherein the ground electrode (120) comprises at least one slot (180, 182) for receiving an end portion (162) of the ground wire (160) for attachment of the ground wire to the ground electrode, and wherein the at least one slot (180) is provided on the edge (142, 144) of the first end portion (122) and/or the second end portion (126) of the ground electrode (120), or wherein the at least one slot (182) is provided in the central portion (124) of the ground electrode.

5. The electrode lead of one of the preceding claims, wherein the first end portion (122) and the second end portion (126) of the ground electrode (120) each are covered by a silicone coating (170, 172) which extends from the outer surface (159) of the tube (150, 152, 154) to the step (134, 136) at the boundary between the central portion (124) and the respective first end portion or the second end portion of the ground electrode so as to seal to the ground electrode to the tube.

6. The electrode lead of claim 5, wherein the outer surface of each of the silicone coatings (170, 172) is substantially flush with the outer surface (128) of the central portion (124) of the ground electrode (120).

7. The electrode lead of one of claims 5 and 6, wherein the first end portion (122) and/or the second end portion (126) of the ground electrode (120) comprises at least one opening (190, 192) for ingress of silicone for anchoring the silicone coating (170, 172) to the respective first or second end portion of the ground electrode.

8. The electrode lead of claim 7, wherein the at least one opening comprises at least one slot (192) provided on the edge (142, 144) of the first end portion (122) and/or the second end portion (126), or wherein the at least one opening comprises at least one hole (190).

9. The electrode lead of one of claims 5 to 8, wherein the outer surface (130, 132) of the first end portion (122) and/or the second end portion (126) of the ground electrode (120) is provided with surface structures (194, 196), such as circumferential ribs or circumferential groves forming a partial thread, for improving adhesion of the silicone coating (170, 172).

10. The electrode lead of one of claims 5 to 9, wherein the outer surface (130, 132) of the first end portion (122) and/or the second end portion (124) of the ground electrode (120) is roughened for improving adhesion of the silicone coating (170, 172).

11. The electrode lead of one of the preceding claims, wherein the tube (150) comprises a first tube portion (152, 152A) and a second tube portion (154, 154A) which are axially aligned to each other and which axially abut each other, thereby forming a circumferential gap (156) in-between which is filled with silicone and which is surrounded by the ground electrode (120).

12. The electrode lead of claim 11, wherein the gap (156) forms the opening of the tube (120) through which the ground wire (160) extends to the ground electrode.

13. A cochlear implant system comprising the electrode lead (110) of one of the preceding claims and a cochlear implant (108) to which the electrode lead is electrically connected, wherein the distance in the axial direction between an end (109) of a housing (111) of the cochlear implant (108) and a central cross sectional plane extending (121) through the center of the axial length of the ground electrode (120) is from 8.8 to 14.8 mm.

14. An electrode lead for a cochlear implant system (100), comprising
a tube (150, 152, 154) having an interior volume (158) and an outer peripheral surface (159);
a plurality of stimulating electrodes (112) disposed on a distal portion of the electrode lead (110);
a ground electrode (220) disposed on a proximal portion of the electrode lead for providing a current return path for stimulation current generated by the plurality of stimulation electrodes, wherein the ground electrode is ring-shaped and is fixed on and around the outer peripheral surface of the tube;
a ground wire (160) extending within the interior volume of the tube to the ground electrode and being electrically connected to the ground electrode through an opening (156) in a wall of the tube;
wherein the ground electrode comprises a first end portion (222), a central portion (224) axially adjacent to the first end portion, and a second end portion (226) axially adjacent to the central portion, and wherein each of the first end portion and the second end portion comprises a ring-like marker structure (223; 227).
